# FASCICULE DE BREVET EUROPEEN

(11) **EP 4 169 900 B1**
(45) Date de publication et mention de la délivrance du brevet: **12.06.2024**
(21) Numéro de dépôt: 22202907.6
(22) Date de dépôt: 21.10.2022
(51) Int. Cl.: C07C 51/41, C07C 63/48, H01M 4/13, H01M 4/139, H01M 4/60

(54) **PROCÉDÉ DE PRÉPARATION D'UN MATÉRIAU DE TYPE CARBOXYLATE MÉTALLIQUE ÉLECTRO-ACTIF**
VERFAHREN ZUR HERSTELLUNG EINES ELEKTROAKTIVEN METALLCARBOXYLATMATERIALS
METHOD FOR PREPARING ELECTROACTIVE METAL CARBOXYLATE MATERIAL

(30) Priorité: 22.10.2021 FR 2111255
(43) Date de publication de la demande: 26.04.2023
(73) Titulaire: Commissariat à l'énergie atomique et aux énergies alternatives, 75015 Paris (FR)
(72) Inventeur: GOUGET, Valentin, 38054 GRENOBLE CEDEX 09 (FR)
(74) Mandataire: Cabinet Nony

(56) Documents cités:
- WO-A1-2006/067109
- CN-A- 108 250 071
- CN-A- 109 301 247
- LIONEL FÉDÈLE ET AL: "Hyper-conjugated lithium carboxylate based on a perylene unit for high-rate organic lithium-ion batteries", JOURNAL OF MATERIALS CHEMISTRY A, vol. 2, no. 43, 18 septembre 2014 (2014-09-18), pages 18225-18228, XP055294388, GB ISSN: 2050-7488, DOI: 10.1039/C4TA02293A
- WANG CHUAN ET AL: "Potassium perylene-tetracarboxylate with two-electron redox behaviors as a highly stable organic anode for K-ion batteries", CHEMICAL COMMUNICATIONS , vol. 55, no. 12 5 février 2019 (2019-02-05), pages 1801-1804, XP055926175, UK ISSN: 1359-7345, DOI: 10.1039/C8CC09596E Extrait de l'Internet: URL:https://pubs.rsc.org/en/content/articl epdf/2019/cc/c8cc09596e

## Description

### Domaine technique

La présente invention vise la synthèse de matériaux de 1a famille des carboxylates métalliques, en particulier des carboxylates de métal alcalin. Elle vise également leur mise en oeuvre comme matériaux actifs d'électrodes organiques, en particulier d'électrodes négatives, dans des systèmes électrochimiques, notamment dans des batteries au lithium.

### Technique antérieure

Les batteries au lithium sont de plus en plus utilisées comme sources d'énergie autonome, notamment dans l'électronique portable. Ces batteries au lithium trouvent en effet de multiples applications, en particulier dans les nouvelles technologies de l'information et de la communication (NTIC), des dispositifs médicaux, les véhicules électriques, le stockage de l'énergie de cellules photovoltaïques, etc.

Pour répondre à des contraintes de coût, de volume de production, d'amélioration de performances en puissance mais aussi de respect à l'égard de l'environnement, des nouveaux matériaux actifs d'électrodes et des nouveaux procédés de préparation de matériaux actifs d'électrode sont en permanence recherchés.

A ce titre, les matériaux d'électrodes organiques se sont révélés particulièrement intéressants pour les systèmes de stockage d'énergie modernes en raison de la durabilité de leurs ressources et de leur respect de l'environnement.

Parmi ces matériaux d'électrodes organiques, les matériaux de type carboxylates métalliques, notamment de type carboxylates de métal alcalin, s'avèrent particulièrement avantageux.

Cependant, ils sont généralement élaborés par un procédé de synthèse conventionnel en milieu aqueux. CN109301247 décrit la réaction du dianhydride de l'acide pérylène-3,4,9,10-tétracarboxylique (PTCDA) avec l'hydroxyde de potassium en solution aqueuse afin de préparer le sel de potassium correspondant. Ce document décrit aussi la préparation d'une électrode avec ledit matériau.

Un tel procédé présente plusieurs inconvénients. En effet, il nécessite une quantité importante d'eau, et génère donc des effluents qui doivent être éliminés dans une étape ultérieure. De plus, les temps de synthèse sont relativement longs et impliquent donc un coût important.

Il existe donc un besoin pour un procédé de synthèse surmontant ces inconvénients.

### Exposé de l'invention

La présente invention a donc précisément pour objet de proposer une nouvelle méthode de préparation d'un matériau de type carboxylate métallique, notamment carboxylate de métal alcalin, permettant de simplifier le procédé et de réduire le temps de synthèse.

En particulier, la présente invention vise à proposer une nouvelle méthode permettant de s'affranchir de l'étape d'élimination des effluents aqueux.

La présente invention vise également à diminuer les coûts de fabrication de ce type de composés.

Plus particulièrement, l'invention concerne, selon un premier de ses aspects, un procédé de préparation d'un matériau de type carboxylate de métal alcalin électro-actif, comprenant au moins les étapes consistant à
(i) disposer d'un mélange solide comprenant au moins un composé source en ions alcalins et au moins un composé aromatique polycondensé choisi parmi les (poly)acides carboxyliques aromatiques polycondensés et les (poly)anhydrides d'acide aromatique polycondensé,
(ii) soumettre ledit mélange solide de l'étape (i) à une contrainte mécanique propice à l'interaction mécanochimique desdits composé(s) source en ions alcalins et composé(s) aromatique(s) polycondensé(s) pour former ledit carboxylate de métal alcalin, et
(iii) récupérer ledit matériau de type carboxylate de métal alcalin, notamment par tamisage. Selon un mode de réalisation préféré, ledit composé aromatique polycondensé est choisi parmi les poly-anhydrides d'acide aromatique polycondensé et les polyacides carboxyliques aromatiques polycondensés, et plus préférentiellement parmi les poly-anhydrides d'acide aromatique polycondensé.

Selon un mode de réalisation particulier, ladite interaction mécanochimique est générée en étape (ii) par broyage mécanique dudit mélange solide.

A la connaissance des inventeurs le procédé selon l'invention n'a jamais été décrit. Certes WO 2006/067109 décrit déjà un procédé pour préparer des carboxylates métalliques par mécanosynthèse, mais ceux-ci ne sont aucunement conformes à ceux considérés selon l'invention, qui sont tout particulièrement intéressants pour leur mise en oeuvre à titre de matériau actif d'électrodes, notamment d'électrodes négatives. De plus, WO 2006/067109 ne concerne ni des carboxylates de métal alcalin ni des composés aromatiques polycondensés.

Comme illustré dans les exemples qui suivent, un tel procédé s'avère particulièrement avantageux à plusieurs titres. Tout d'abord, la synthèse peut avoir lieu en voie solide, et ne nécessite pas l'ajout de solvant et en particulier d'eau dans le milieu de synthèse. De plus, ce procédé ne génère pas d'effluents, notamment aqueux. Il permet donc avantageusement de s'affranchir d'une étape de séparation des effluents aqueux en fin de synthèse.

De plus, le procédé selon l'invention permet d'atteindre des temps de synthèse plus courts que ceux de l'art antérieur. En particulier, la synthèse peut être réalisée en une heure, alors qu'elle dure 24h pour un procédé conventionnel. Par ailleurs, le procédé selon l'invention permet d'atteindre un rendement élevé en carboxylate métallique, notamment en carboxylate de métal alcalin.

Enfin, le procédé selon l'invention est de mise en oeuvre aisée au moyen d'un broyeur conventionnel, pourvu que le broyeur apporte une énergie de broyage suffisante pour que la réaction du mélange de l'étape (i) puisse s'opérer.

Les inventeurs ont donc constaté qu'il est possible d'accéder *via* un tel procédé, à un matériau de type carboxylate métallique, notamment de type carboxylate de métal alcalin, particulièrement avantageux à titre de matériau actif d'électrodes organiques, notamment d'électrodes négatives, et en particulier pour des batteries secondaires au lithium.

Ainsi, l'invention concerne également un matériau de type carboxylate de métal alcalin, notamment particulaire, obtenu par le procédé selon l'invention tel que défini précédemment.

Elle concerne également, selon un autre de ses aspects, l'utilisation d'un matériau de type carboxylate de métal alcalin obtenu par le procédé de l'invention, à titre de matériau actif d'électrode, en particulier d'électrode négative.

Elle concerne encore une électrode comprenant, à titre de matériau actif, au moins un matériau de type carboxylate de métal alcalin obtenu par le procédé de l'invention.

Elle concerne également un procédé de préparation d'une telle électrode comprenant au moins les étapes suivantes consistant à :
(a) disposer d'une dispersion comprenant, dans un milieu aqueux ou organique, au moins un matériau de type carboxylate de métal alcalin obtenu selon l'invention, et éventuellement un ou plusieurs additif(s) conducteur(s) électronique(s) et/ou liant(s) ;
(b) procéder au dépôt de tout ou partie de ladite dispersion (a) à la surface d'un collecteur de courant, par exemple de type feuillard en aluminium ou feutre de carbone ; et
(c) évaporer ledit milieu aqueux ou organique pour former ladite électrode.

En particulier, elle concerne un procédé de préparation d'une électrode comprenant, à titre de matériau actif, au moins un matériau de type carboxylate de métal alcalin, notamment d'une électrode selon l'invention, comprenant au moins les étapes suivantes consistant à :
(i) disposer d'un mélange solide comprenant au moins un composé source en ions alcalins et au moins un composé aromatique polycondensé choisi parmi les (poly)acides carboxyliques aromatiques polycondensés et les (poly)anhydrides d'acide aromatique polycondensé ;
(ii) soumettre ledit mélange solide de l'étape (i) à une contrainte mécanique propice à l'interaction mécanochimique desdits composé(s) source en ions alcalins et composé(s) aromatique(s) polycondensé(s) pour former ledit carboxylate de métal alcalin ;
(iii) récupérer ledit matériau de type carboxylate de métal alcalin, notamment par tamisage ;
(iv) préparer une dispersion comprenant, dans un milieu aqueux ou organique, au moins tout ou partie dudit matériau de type carboxylate de métal alcalin de l'étape (iii), et éventuellement un ou plusieurs additif(s) conducteur(s) électronique(s) et/ou liant(s) ;
(v) procéder au dépôt de tout ou partie de ladite dispersion de l'étape (iv) à la surface d'un collecteur de courant, par exemple de type feuillard en aluminium ou feutre de carbone ; et
(vi) évaporer ledit milieu aqueux ou organique pour former ladite électrode.

L'invention concerne encore, selon un autre de ses aspects, un système électrochimique comprenant au moins une électrode selon l'invention ou obtenue par le procédé selon l'invention, notamment une électrode négative.

Un tel système électrochimique est plus particulièrement une batterie organique, en particulier une batterie en configuration cation-ion, notamment une batterie lithium-ion, sodium-ion, potassium-ion, magnésium-ion, une batterie en configuration « dual-ion », dans laquelle à la fois des cations et des anions sont impliqués dans la réaction rédox électrochimique (anode métallique ou non).

D'autres caractéristiques, variantes et avantages des objets de l'invention, ressortiront mieux à la lecture de la description, des exemples et figures qui vont suivre, donnés à titre illustratif et non limitatif de l'invention.

Dans la suite du texte, les expressions « compris entre ... et ... », « allant de ... à ... » et « variant de ... à ... » sont équivalentes et entendent signifier que les bornes sont incluses, sauf mention contraire.

### Brève description des dessins

[Fig 1] représente un spectre d'analyse IR à transformation de Fourier (IR-ATF) d'un matériau de type carboxylate de métal alcalin conforme à l'invention préparé en exemple.
[Fig 2] représente un diagramme de diffraction aux rayons X (XRD) d'un matériau de type carboxylate de métal alcalin conforme à l'invention préparé en exemple.

### Description détaillée

### Préparation d'un matériau de type carboxylate de métal alcalin électro-actif

### Etape (i)

Comme mentionné précédemment, l'étape (i) du procédé de l'invention consiste à disposer d'un mélange solide comprenant au moins un composé source en ions alcalins et au moins un composé aromatique polycondensé choisi parmi les (poly)acides carboxyliques aromatiques polycondensés et les (poly)anhydrides d'acide aromatique polycondensé.

### Composé source en ions alcalins

Au sens de l'invention, un composé source en ions alcalins est un composé apte à générer des ions alcalins. Plus particulièrement, le composé source en ions alcalins est apte à réagir avec une fonction acide carboxylique ou anhydride d'acide carboxylique pour former un carboxylate de métal alcalin.

Le composé source en ions alcalins peut comprendre un ou plusieurs ions alcalins identiques ou différents, de préférence identiques. En particulier, il comprend un unique métal alcalin.

En particulier, le composé source en ions alcalins est choisi parmi les composés source en ions lithium, sodium, potassium et leurs mélanges. De préférence, le mélange solide comprend au moins un composé source en ions lithium.

Le composé source en ions alcalins peut être choisi parmi les composés inorganiques source en ions alcalins, et les composés organiques source en ions alcalins.

Les composés inorganiques source en ions alcalins peuvent être choisis parmi les hydroxydes de métal alcalin, les carbonates de métal alcalin, les hydrures de métal alcalins et leurs mélanges, en particulier parmi les hydroxydes de métal alcalin, les carbonates de métal alcalin et leurs mélanges. De préférence, le mélange solide en étape (i) comprend au moins un hydroxyde de métal alcalin.

A titre d'exemples de composés inorganiques source en ions alcalins peuvent être cités LiOH, Li₂CO₃, LiH, NaOH, Na₂CO₃, NaH, KOH, K₂CO₃ et KH.

Les composés inorganiques source en ions alcalins peuvent être sous une forme hydratée ou anhydre, notamment hydratée.

Selon un mode de réalisation préféré, le composé source en ions alcalins est un composé inorganique source en ions alcalins. En particulier, le mélange solide de l'étape (i) comprend au moins un composé source en ions alcalins choisi parmi les carbonates de lithium, sodium ou potassium, et les hydroxydes de lithium, sodium ou potassium et leurs mélanges, de préférence choisi parmi les hydroxydes de lithium, sodium ou potassium et plus préférentiellement l'hydroxyde de lithium. Selon un mode de réalisation préféré, le composé source en ions alcalins est l'hydroxyde de lithium.

### Composé aromatique polycondensé

Au sens de l'invention, le terme « aromatique polycondensé » désigne une structure chimique comprenant plusieurs cycles aromatiques condensés avec certains des atomes de carbone étant communs à deux ou trois cycles. Une structure de ce type est également appelée hydrocarbure à noyaux condensés ou hydrocarbure aromatique polycyclique (HAP). Les cycles peuvent être alignés ou présenter une disposition angulaire ou compacte.

Les structures aromatiques polycondensées convenant à l'invention sont de préférence en C10 à C30, plus préférentiellement en C15 à C25. En particulier, elles peuvent posséder au moins deux, trois, quatre ou cinq cycles condensés. Ces cycles peuvent être substitués ou non notamment par un ou plusieurs radicaux alkyles en C1 à C6, linéaires ou ramifiés. Ils peuvent contenir un ou plusieurs hétéroatomes notamment choisis parmi les atomes d'oxygène, de soufre, de bore et d'azote.

En particulier, les structures aromatiques polycondensées sont non substituées. En particulier, elles ne contiennent pas d'hétéroatomes.

Dans le cadre de l'invention, les composés aromatiques polycondensés considérés sont choisis parmi les monoacides carboxyliques aromatiques polycondensés, les polyacides carboxyliques aromatiques polycondensés, les mono-anhydrides d'acide aromatique polycondensé et les poly-anhydrides d'acide aromatique polycondensé. De préférence, ils sont choisis parmi les polyacides carboxyliques aromatiques polycondensés et les poly-anhydrides d'acide aromatique polycondensé.

Par mono-anhydride d'acide, on entend un composé comprenant une unique fonction anhydride d'acide carboxylique. Par monoacide, on entend un composé comprenant une unique fonction acide carboxylique.

Par poly-anhydride d'acide, on entend un composé comprenant au moins deux fonctions anhydride d'acide carboxylique. Par polyacide, on entend un composé comprenant au moins deux fonctions acide carboxylique.

En particulier, les composés aromatiques polycondensés considérés sont des composés comprenant au moins une fonction anhydride d'acide carboxylique et/ou au moins deux fonctions acide carboxylique. Plus particulièrement, les composés aromatiques polycondensés considérés comprennent au moins deux fonctions anhydride d'acide carboxylique, ou au moins quatre fonctions acide carboxylique, ou au moins une fonction anhydride d'acide carboxylique et au moins deux fonctions acide carboxylique.

De préférence, le mélange de l'étape (i) comprend au moins un composé aromatique polycondensé choisi parmi les di-anhydrides d'acide tétracarboxylique aromatique polycondensé et les acides tétracarboxyliques aromatiques polycondensés et de préférence parmi les di-anhydrides d'acide tétracarboxylique aromatique polycondensé.

Par di-anhydride d'acide, on entend un composé comprenant deux fonctions anhydride d'acide carboxylique. Par acide tétracarboxylique, on entend un composé comprenant quatre fonctions acide carboxylique.

En particulier, le composé aromatique polycondensé est dénué de fonction hydroxyle.

A titre illustratif des di-anhydrides d'acide tétracarboxylique aromatique polycondensé et des acides tétracarboxyliques aromatiques polycondensés convenant à l'invention peuvent notamment être cités les di-anhydrides d'acide tétracarboxylique et les acides tétracarboxyliques, notamment les di-anhydrides d'acide tétracarboxylique, dont la structure aromatique polycondensée dérive de l'anthracène, du benzoanthracène, du benzofluoranthène, du 1,12-benzopérylène, du benzopyrène, du chrysène, du dibenzoacridine, du dibenzoanthracène, du dibenzofurane, du dibenzopyrène, du pérylène ou du phénanthrène, de préférence non hydroxylés.

Plus particulièrement, le di-anhydride d'acide aromatique polycondensé est choisi parmi le di-anhydride de l'acide 1,2,5,6-naphthalènetétracarboxylique, le di-anhydride de l'acide 2,3,6,7-naphthalènetétracarboxylique, le di-anhydride de l'acide 3,4,9,10-pérylènetétracarboxylique (dit PTCDA) et leurs mélanges.

Plus particulièrement, l'acide tétracarboxylique aromatique polycondensé est choisi parmi l'acide 1,2,5,6- naphthalènetétracarboxylique, l'acide 2,3,6,7-naphthalènetétracarboxylique, l'acide 3,4,9,10-pérylènetétracarboxylique et leurs mélanges.

Selon un mode préféré de l'invention, le mélange de l'étape (i) comprend au moins un composé aromatique polycondensé choisi parmi le di-anhydride de l'acide 3,4,9,10-pérylènetétracarboxylique, l'acide 3,4,9,10-pérylènetétracarboxylique et leur mélange. Selon un mode particulièrement préféré de l'invention, le mélange de l'étape (i) comprend au moins le di-anhydride de l'acide 3,4,9,10-pérylènetétracarboxylique de formule 1 ci-dessous.

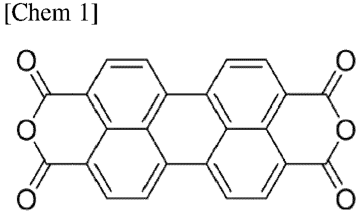

### Mélange solide

Le mélange solide en étape (i) comprend au moins un composé source en ions alcalins et au moins un composé aromatique polycondensé choisi parmi les (poly)acides carboxyliques aromatiques polycondensés et les (poly)anhydrides d'acide aromatique polycondensé.

Par mélange solide, on entend que tous les composés du mélange sont à l'état solide, en particulier à température ambiante, notamment à une température allant de 15 °C à 25°C, et que le mélange est dénué d'un milieu solvant liquide, par exemple dans lequel ces composés seraient en dispersion ou en solution.

En particulier, le mélange solide de l'étape (i) est un mélange particulaire, notamment sous la forme d'une poudre.

Le composé source en ions alcalins est avantageusement un composé solide dans les conditions de synthèse de l'étape (ii). De préférence, il est solide à température ambiante, notamment à une température allant de 15 °C à 25°C.

Selon un mode de réalisation préféré, le composé source en ions alcalins est présent dans le mélange solide sous la forme d'une poudre.

Le composé aromatique polycondensé est avantageusement un composé solide dans les conditions de synthèse de l'étape (ii). De préférence, il est solide à température ambiante. En particulier, le composé aromatique polycondensé possède une température de dégradation ou de fusion supérieure ou égale à 100°C, de préférence supérieure ou égale à 220°C voire supérieure ou égale à 280°C.

Selon un mode de réalisation préféré, le composé aromatique polycondensé est présent dans le mélange solide sous la forme d'une poudre.

Les tailles des particules des poudres des composé(s) source en ions alcalins et composé(s) aromatique(s) polycondensé(s) peuvent avantageusement être sélectionnées pour améliorer l'efficacité de la réaction du ou des composé(s) aromatique(s) polycondensé(s) avec le ou les composé(s) source en ions alcalins au cours de l'étape (ii).

Selon un mode de réalisation particulier, le(s) composé(s) aromatique(s) polycondensé(s) et le(s) composé(s) source(s) en ions alcalins sont mis en oeuvre dans un rapport molaire composé(s) aromatique(s) polycondensé(s)/composé(s) source(s) en ions alcalins variant de 0,05 à 1, de préférence de 0,1 à 0,5, et plus préférentiellement de 0,2 à 0,3.

Comme précisé ci-dessus le mélange solide est dénué d'un milieu solvant liquide et en particulier d'eau libre.

L'eau libre est à distinguer de l'eau présente sous forme solvatée dans les composés du mélange solide, ou de l'eau présente dans leur structure chimique. En particulier, l'eau libre est à distinguer de l'eau présente dans un composé inorganique source en ions alcalins sous forme hydratée, par exemple dans l'hydroxyde de lithium monohydrate. En particulier, l'eau libre peut être distinguée de l'eau adsorbée provenant de l'humidité ambiante.

En particulier, le mélange solide est avantageusement sous la forme d'un mélange particulaire, notamment sous la forme d'un mélange sec particulaire.

Selon un mode de réalisation particulier, le mélange solide est constitué, pour au moins 90,0 % de sa masse, de préférence au moins 95,0 % de sa masse, voire au moins 97,0 % de sa masse, voire au moins 99,0 % de sa masse, d'un ou plusieurs composé(s) source(s) en ions alcalins, en particulier lithium, et d'un ou plusieurs composé(s) aromatique(s) polycondensé(s) choisi(s) parmi les (poly)acides carboxyliques aromatiques polycondensés et les (poly)anhydrides d'acide aromatique polycondensé.

Selon un mode de réalisation particulier, le mélange solide est constitué, pour au moins 90,0 % de sa masse, de préférence au moins 95,0 % de sa masse, voire au moins 97,0 % de sa masse, voire au moins 99,0 % de sa masse, d'un ou plusieurs hydroxydes de lithium, sodium ou potassium, en particulier de lithium, et d'un di-anhydride d'acide tétracarboxylique aromatique polycondensé et/ou d'un acide tétracarboxylique aromatique polycondensé notamment choisi(s) parmi le di-anhydride de l'acide 3,4,9,10-pérylènetétracarboxylique et l'acide 3,4,9,10-pérylènetétracarboxylique, en particulier le dianhydride de l'acide 3,4,9,10-pérylènetétracarboxylique.

Selon un mode de réalisation préféré, le mélange solide de l'étape (i) comprend au moins l'hydroxyde de lithium anhydre ou hydraté, notamment hydraté, et au moins le di-anhydride de l'acide 3,4,9,10-pérylènetétracarboxylique.

### Etape (ii)

L'étape (ii) du procédé de l'invention consiste à soumettre ledit mélange solide de l'étape (i) à une contrainte mécanique propice à l'interaction mécanochimique desdits composé(s) source en ions alcalins et composé(s) aromatique(s) polycondensé(s) pour former ledit carboxylate de métal alcalin.

Comme précisé ci-dessus, ledit mélange de l'étape (i), soumis à la contrainte mécanique, est dénué d'un milieu liquide et en particulier d'eau libre.

Avantageusement, l'interaction mécanochimique est générée en étape (ii) par broyage mécanique dudit mélange solide de manière à former le carboxylate de métal alcalin.

En particulier, le mélange est broyé en l'absence d'un milieu liquide, et plus particulièrement en l'absence d'eau libre.

L'étape de broyage du mélange solide de l'étape (i) peut être mise en oeuvre au sein d'un broyeur. Par exemple, le broyage peut être réalisé dans un broyeur planétaire ou un broyeur à billes, en particulier dans un broyeur planétaire. L'invention n'est toutefois pas limitée à ces types de broyeur. Tout autre broyeur conventionnel peut être utilisé, pourvu que l'énergie de broyage qu'il est apte à apporter au mélange solide soit suffisante pour que les constituants du mélange solide réagissent ensemble.

En particulier, le broyage est réalisé à une vitesse allant de 100 rpm à 1000 rpm. Il peut être réalisé pendant une durée de 30 min à 7 h, de préférence de 30 min à 2 h. Il peut être réalisé dans un bol de broyage en agate et/ou avec des billes en agate.

A l'issue du broyage, on obtient avantageusement un carboxylate de métal alcalin, de préférence sous la forme d'une poudre, dont les particules présentent en particulier une taille médiane allant de 0,2 à 45 µm, notamment allant de 1 à 10 µm.

La « taille » d'une particule est sa plus grande dimension, par exemple observable sur un cliché pris au moyen d'un microscope électronique à balayage, suivant une direction perpendiculaire au plan sur lequel repose ladite particule.

En particulier, le carboxylate de métal alcalin formé est un composé aromatique polycondensé comprenant au moins une fonction carboxylate de métal alcalin, de préférence au moins quatre fonctions carboxylate de métal alcalin, et plus préférentiellement comprenant quatre fonctions carboxylate de métal alcalin, notamment de lithium. Plus particulièrement, le carboxylate de métal alcalin formé est un 3,4,9,10-pérylènetétracarboxylate de métal alcalin, notamment le 3,4,9,10-pérylènetétracarboxylate de lithium.

L'étape (ii) peut notamment permettre d'adapter la granulométrie de la poudre obtenue.

### Etape (iii)

L'étape (iii) du procédé de l'invention consiste à récupérer ledit matériau de type carboxylate de métal alcalin.

Avantageusement, le matériau de type carboxylate de métal alcalin est récupéré par tamisage.

Ainsi, l'invention concerne également un matériau de type carboxylate de métal alcalin, notamment particulaire, obtenu par le procédé tel que défini précédemment.

Le matériau de type carboxylate de métal alcalin selon l'invention possède avantageusement une taille médiane de particules comprise entre 0,2 et 45 µm, de préférence entre 1 et 10 µm.

### Utilisation comme matériau actif d'électrode

Comme évoqué précédemment, le matériau de type carboxylate de métal alcalin selon l'invention peut avantageusement être mis en oeuvre comme matériau actif d'électrode organique.

Par « matériau ou composé actif d'électrode », on entend au sens de l'invention un matériau (respectivement un composé) d'insertion/désinsertion d'un cation Cⁿ⁺, dans lequel n vaut 1 ou 2 (Li⁺, Na⁺, K⁺, Ca²⁺ ou Mg²⁺), d'une électrode d'un générateur électrochimique. Plus particulièrement, le matériau (composé) actif de l'électrode positive est capable de libérer des ions Cⁿ⁺ au moment de la charge et d'incorporer des ions Cⁿ⁺ au moment de la décharge du générateur électrochimique. Inversement, le matériau (composé) actif de l'électrode négative est capable d'incorporer des ions Cⁿ⁺ au moment de la charge et de libérer des ions Cⁿ⁺ au moment de la décharge du générateur électrochimique.

Ainsi, l'invention concerne encore l'utilisation d'un matériau de type carboxylate de métal alcalin selon l'invention ou tel qu'obtenu par le procédé de l'invention, à titre de matériau actif d'électrode, en particulier d'électrode négative. De préférence, il est utilisé comme matériau actif d'électrode, notamment négative, en particulier pour un générateur électrochimique, notamment pour une batterie secondaire au lithium, au sodium, au potassium ou au magnésium, de préférence au lithium.

L'invention a également trait à une électrode comprenant, à titre de matériau actif, au moins un matériau de type carboxylate de métal alcalin selon l'invention.

De préférence, le ou les matériau(x) de type carboxylate de métal alcalin, mis en oeuvre comme matériau actif d'électrode selon l'invention, représente(nt) avantageusement plus de 60% massique de la masse totale de l'électrode, en particulier de 70 % à 99 % massique, et plus particulièrement de 80 à 96 % massique de la masse totale de l'électrode.

Le ou lesdits matériau(x) de type carboxylate de métal alcalin selon l'invention peu(ven)t être mis en oeuvre, de manière classique, conjointement avec un ou plusieurs liant(s), en particulier un ou plusieurs liants polymériques.

De tels liants peuvent être choisis parmi des liants fluorés, en particulier parmi le polytétrafluoroéthylène, le polyfluorure de vinylidène (PVDF), la carboxyméthylcellulose et ses dérivés, les polysaccharides, les polyacrylates, les latex notamment de type styrènebutadiène.

De préférence, le liant mis en oeuvre est le polyfluorure de vinylidène (PVDF).

En particulier, le ou lesdits liants peuvent être mis en oeuvre au niveau de la composition de l'électrode organique, à raison de 0,5 % à 20 % massique, en particulier de 1 % à 10 % massique, voire de 1 % à 5 % massique par rapport à la masse totale de l'électrode.

Le ou lesdits matériau(x) de type carboxylate de métal alcalin selon l'invention peuvent être mis en oeuvre, de manière classique, conjointement avec un ou plusieurs additif(s) conducteur(s) électronique(s).

Le ou lesdits additifs conducteurs électroniques peuvent être choisis parmi des fibres de carbone, du noir de carbone, des nanotubes de carbone, du graphène, du graphite, et des particules métalliques, tels que des nanofils ou nanoparticules métalliques.

Les particules métalliques peuvent être par exemple en aluminium, en cuivre ou en nickel. De préférence, les additifs conducteurs électroniques sont choisis parmi des additifs carbonés, notamment des nanofibres de carbone et du noir de carbone.

Le ou lesdits additifs conducteurs électroniques, en particulier carbonés, peuvent être mis en oeuvre au niveau de la composition de l'électrode organique, à raison de 0,5 % à 20 % massique, en particulier de 1 % à 10 % massique, voire de 1 % à 5 % massique par rapport à la masse totale de l'électrode.

L'électrode à base du ou desdits matériau(x) de type carboxylate de métal alcalin selon l'invention peut ainsi comprendre, outre le ou lesdits matériau(x) de type carboxylate de métal alcalin, un ou plusieurs additif(s) conducteur(s) électronique(s) et/ou un ou plusieurs liant(s), en particulier tels que décrits précédemment.

Selon un mode de réalisation particulier, une électrode organique selon l'invention peut ainsi comprendre, voire être formée :
- de 60 à 99 % massique, en particulier de 80 à 99 % massique, notamment de 90 à 95 % massique d'un ou plusieurs matériau(x) de type carboxylate de métal alcalin préparé(s) selon l'invention ;
- de 0,5 à 20 % massique, en particulier de 1 à 10 % massique et de préférence de 1 à 5 % massique d'un ou plusieurs additif(s) conducteur(s) électronique(s), en particulier de type carboné ; et
- de 0,5 à 20 % massique, en particulier de 1 à 10 % massique et de préférence de 1 à 5 % massique d'un ou plusieurs liants, en particulier de polyfluorure de vinylidène ;
les pourcentages étant exprimés par rapport à la masse totale de l'électrode (hors collecteur de courant).

L'électrode, comprenant en tant que matière électro-chimiquement active, un matériau de type carboxylate de métal alcalin selon l'invention, peut être une électrode positive ou une électrode négative, de préférence une électrode négative.

De manière classique, l'électrode est en contact avec un collecteur de courant.

Par exemple, du cuivre, de l'aluminium, du nickel, du feutre de carbone ou de l'acier inoxydable peuvent être utilisés comme collecteur de courant pour une électrode positive ; et du cuivre, ou de l'acier, traités en une feuille découpée, un métal en mousse ou une plaque de feuille laminée, par exemple, peuvent être utilisés comme collecteur de courant pour une électrode négative.

Selon un mode de réalisation particulier, une électrode selon l'invention comprend un collecteur de courant à base d'aluminium ou de carbone, par exemple sous la forme d'une feuille d'aluminium ou d'un feutre de carbone, de préférence sous la forme d'une feuille d'aluminium.

### Préparation de l'électrode organique

Pour la préparation de l'électrode, le matériau de type carboxylate de métal alcalin selon l'invention est avantageusement formulé avec un ou plusieurs solvants aqueux ou organiques.

Ainsi, une électrode organique à base d'un matériau de type carboxylate de métal alcalin selon l'invention, peut être préparée *via* au moins les étapes suivantes :
(a) disposer d'une dispersion, couramment appelée « encre », comprenant, dans un milieu aqueux ou organique, au moins un matériau de type carboxylate de métal alcalin obtenu selon l'invention, et éventuellement un ou plusieurs additif(s) conducteur(s) électronique(s) et/ou liant(s) ;
(b) procéder au dépôt de tout ou partie de ladite dispersion (a) à la surface d'un collecteur de courant ; et
(c) évaporer ledit milieu aqueux ou organique pour former ladite électrode.

Ainsi, l'invention concerne encore une encre utile pour la préparation d'une électrode organique, comprenant, dans un milieu aqueux ou organique, au moins un matériau de type carboxylate de métal alcalin selon l'invention, et éventuellement un ou plusieurs additif(s) conducteur(s) électronique(s) et/ou liant(s).

### Système électrochimique

L'invention concerne également un système électrochimique comprenant au moins une électrode organique comprenant un matériau de type carboxylate de métal alcalin obtenu selon l'invention.

Le système électrochimique dans lequel est mis en oeuvre l'électrode selon l'invention peut être notamment un accumulateur électrochimique rechargeable, en particulier un accumulateur ou une batterie au lithium.

De préférence, cet accumulateur électrochimique rechargeable comprend, outre l'électrode organique positive ou négative telle que définie précédemment comprenant un matériau de type carboxylate de métal alcalin selon l'invention, une électrode négative ou positive ne comprenant pas un matériau de type carboxylate de métal alcalin selon l'invention, et un électrolyte.

L'invention va maintenant être décrite au moyen des figures et exemples suivants, donnés bien entendu à titre illustratif et non limitatif de l'invention.

### Exemple

La réaction de synthèse du carboxylate de lithium est réalisée selon le schéma suivant :

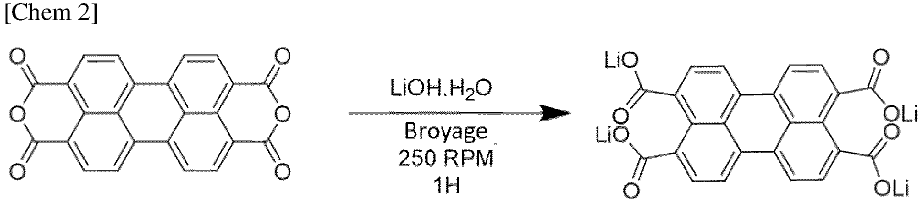

Tout d'abord, 2 g de PTCDA (Sigma-Aldrich) et 913 mg de LiOH.H₂O (Sigma-Aldrich) sont placés dans un bol de broyage de 50 ml en agate. 45g de billes en agate de diamètre 6 mm sont ajoutées au bol de broyage. Le mélange est broyé à 250 rpm pendant une heure dans un broyeur planétaire commercialisé sous la dénomination PM 100 par la société Retsch. Après tamisage sur un tamis de maille 5 mm, une poudre de carboxylate de lithium est récupérée.

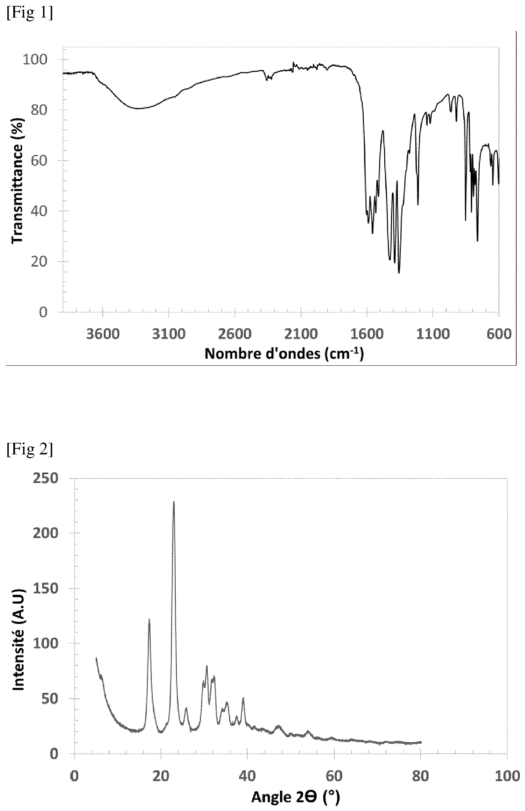

Le produit obtenu a été caractérisé par analyse IR à transformation de Fournier (IR-ATF) comme présenté en figure 1 et par diffractométrie aux rayons X (XRD) comme présenté en figure 2.

La réaction a un rendement de 88,1%.

## Revendications

1. Procédé de préparation d'un matériau de type carboxylate de métal alcalin électro-actif, comprenant au moins les étapes consistant à
(i) disposer d'un mélange solide comprenant au moins un composé source en ions alcalins et au moins un composé aromatique polycondensé choisi parmi les (poly)acides carboxyliques aromatiques polycondensés et les (poly)anhydrides d'acide aromatique polycondensé,
(ii) soumettre ledit mélange solide de l'étape (i) à une contrainte mécanique propice à l'interaction mécanochimique desdits composé(s) source en ions alcalins et composé(s) aromatique(s) polycondensé(s) pour former ledit carboxylate de métal alcalin, et
(iii) récupérer ledit matériau de type carboxylate de métal alcalin, notamment par tamisage.

2. Procédé selon la revendication 1, dans lequel ladite interaction mécanochimique est générée en étape (ii) par broyage mécanique dudit mélange solide, en particulier dans un broyeur planétaire ou un broyeur à billes, notamment dans un broyeur planétaire.

3. Procédé selon l'une quelconque des revendications précédentes, dans lequel ledit mélange solide de l'étape (i), soumis à la contrainte mécanique, est dénué d'un milieu liquide et en particulier d'eau libre.

4. Procédé selon l'une quelconque des revendications précédentes, dans lequel ledit mélange solide de l'étape (i) est un mélange particulaire.

5. Procédé selon l'une quelconque des revendications précédentes, dans lequel ledit mélange solide de l'étape (i) comprend au moins un composé source en ions alcalins choisi parmi les composés source en ions lithium, sodium, potassium et leurs mélanges, et de préférence comprend au moins un composé source en ions lithium.

6. Procédé selon l'une quelconque des revendications précédentes, dans lequel ledit mélange solide de l'étape (i) comprend au moins un composé source en ions alcalins choisi parmi les carbonates de lithium, sodium ou potassium, et les hydroxydes de lithium, sodium ou potassium, et leurs mélanges, de préférence choisi parmi les hydroxydes de lithium, sodium ou potassium et plus préférentiellement l'hydroxyde de lithium.

7. Procédé selon l'une quelconque des revendications précédentes, dans lequel le mélange solide de l'étape (i) comprend au moins un composé aromatique polycondensé choisi parmi les polyacides carboxyliques aromatiques polycondensés et les poly-anhydrides d'acide aromatique polycondensé, de préférence parmi les di-anhydrides d'acide tétracarboxylique aromatique polycondensé et les acides tétracarboxyliques aromatiques polycondensés, et plus préférentiellement parmi les di-anhydrides d'acide tétracarboxylique aromatique polycondensé.

8. Procédé selon l'une quelconque des revendications précédentes, dans lequel ledit composé aromatique polycondensé est dénué de fonction hydroxyle.

9. Procédé selon l'une quelconque des revendications précédentes, dans lequel ledit mélange solide de l'étape (i) comprend au moins un composé aromatique polycondensé choisi parmi les di-anhydrides d'acide tétracarboxylique et les acides tétracarboxyliques, notamment les di-anhydrides d'acide tétracarboxylique, dont la structure aromatique polycondensée dérive de l'anthracène, du benzoanthracène, du benzofluoranthène, du 1,12-benzopérylène, du benzopyrène, du chrysène, du dibenzoacridine, du dibenzoanthracène, du dibenzofurane, du dibenzopyrène, du pérylène ou du phénanthrène, de préférence choisi parmi le di-anhydride de l'acide 3,4,9,10-pérylènetétracarboxylique, l'acide 3,4,9,10-pérylènetétracarboxylique et leur mélange, et plus préférentiellement comprend au moins le di-anhydride de l'acide 3,4,9,10-pérylènetétracarboxylique.

10. Procédé selon l'une quelconque des revendications précédentes, dans lequel le mélange solide de l'étape (i) comprend au moins l'hydroxyde de lithium anhydre ou hydraté, notamment hydraté, et au moins le di-anhydride de l'acide 3,4,9,10-pérylènetétracarboxylique.

11. Procédé de préparation d'une électrode comprenant, à titre de matériau actif, au moins un matériau de type carboxylate de métal alcalin, comprenant au moins les étapes suivantes consistant à :
(i) disposer d'un mélange solide comprenant au moins un composé source en ions alcalins et au moins un composé aromatique polycondensé choisi parmi les (poly)acides carboxyliques aromatiques polycondensés et les (poly)anhydrides d'acide aromatique polycondensé ;
(ii) soumettre ledit mélange solide de l'étape (i) à une contrainte mécanique propice à l'interaction mécanochimique desdits composé(s) source en ions alcalins et composé(s) aromatique(s) polycondensé(s) pour former ledit carboxylate de métal alcalin ;
(iii) récupérer ledit matériau de type carboxylate de métal alcalin, notamment par tamisage ;
(iv) préparer une dispersion comprenant, dans un milieu aqueux ou organique, au moins tout ou partie dudit matériau de type carboxylate de métal alcalin de l'étape (iii), et éventuellement un ou plusieurs additif(s) conducteur(s) électronique(s) et/ou liant(s) ;
(v) procéder au dépôt de tout ou partie de ladite dispersion de l'étape (iv) à la surface d'un collecteur de courant ; et
(vi) évaporer ledit milieu aqueux ou organique pour former ladite électrode.

12. Procédé selon la revendication précédente, dans lequel les étapes (i), (ii) et (iii) sont telles que définies dans l'une quelconque des revendications 1 à 10.

## Patentansprüche

1. Verfahren zur Herstellung eines Materials vom Typ elektroaktives Alkalimetallcarboxylat, wobei es zumindest die folgenden Schritte umfasst
(i) Bereitstellen einer feststofflichen Mischung, die mindestens eine Verbindung, welche eine Quelle von Alkaliionen ist, und mindestens eine polykondensierte aromatische Verbindung umfasst, welche aus den polykondensierten aromatischen (Poly)carbonsäuren und den (Poly)anhydriden polykondensierter aromatischer Säure ausgewählt ist,
(ii) Einwirkenlassen einer mechanischen Belastung, welche eine mechanochemische Wechselwirkung zwischen der/den Verbindung(en), welche eine Quelle von Alkaliionen ist/sind, und der/den polykondensierten aromatischen Verbindung(en) begünstigt, auf die feststoffliche Mischung des Schrittes (i), um das Alkalimetallcarboxylat zu bilden, und
(iii) Gewinnen des Materials vom Typ Alkalimetallcarboxylat, insbesondere durch Sieben.

2. Verfahren nach Anspruch 1, wobei die mechanochemische Wechselwirkung im Schritt (ii) durch mechanisches Vermahlen der feststofflichen Mischung bewirkt wird, besonders in einer Planetenmühle oder einer Kugelmühle, insbesondere in einer Planetenmühle.

3. Verfahren nach einem beliebigen der vorhergehenden Ansprüche, wobei die feststoffliche Mischung des Schrittes (i), auf welche die mechanische Belastung einwirkt, frei von jedwedem flüssigen Medium, insbesondere von ungebundenem Wasser ist.

4. Verfahren nach einem beliebigen der vorhergehenden Ansprüche, wobei es sich bei der feststofflichen Mischung des Schrittes (i) um eine partikelförmige Mischung handelt.

5. Verfahren nach einem beliebigen der vorhergehenden Ansprüche, wobei die feststoffliche Mischung des Schrittes (i) mindestens eine Verbindung umfasst, welche eine Quelle von Alkaliionen ist, wobei sie aus den Verbindungen ausgewählt ist, die eine Quelle von Lithium, Natrium-, Kaliumionen und von deren Mischungen ist, wobei sie vorzugsweise mindestens eine Verbindung umfasst, die eine Quelle von Lithiumionen ist.

6. Verfahren nach einem beliebigen der vorhergehenden Ansprüche, wobei die feststoffliche Mischung des Schrittes (i) mindestens eine Verbindung umfasst, die eine Quelle von Alkaliionen ist, welche aus den Carbonaten von Lithium, Natrium oder Kalium und den Hydroxiden von Lithium, Natrium oder Kalium sowie deren Mischungen ausgewählt ist, wobei sie vorzugsweise aus den Hydroxiden von Lithium, Natrium oder Kalium ausgewählt ist und es sich stärker bevorzugt um Lithiumhydroxid handelt.

7. Verfahren nach einem beliebigen der vorhergehenden Ansprüche, wobei die feststoffliche Mischung des Schrittes (i) mindestens eine polykondensierte aromatische Verbindung umfasst, die aus den polykondensierten aromatischen Polycarbonsäuren und den Polyanhydriden polykondensierter aromatischer Säure, vorzugsweise aus den Dianhydriden polykondensierter aromatischer Tetracarbonsäure und den polykondensierten aromatischen Tetracarbonsäuren ausgewählt ist, und stärker bevorzugt aus den Dianhydriden polykondensierter aromatischer Tetracarbonsäure.

8. Verfahren nach einem beliebigen der vorhergehenden Ansprüche, wobei die polykondensierte aromatische Verbindung über keinerlei funktionelle Hydroxylgruppen verfügt.

9. Verfahren nach einem beliebigen der vorhergehenden Ansprüche, wobei die feststoffliche Mischung des Schrittes (i) mindestens eine polykondensierte aromatische Verbindung umfasst, die aus den Tetracarbonsäuredianhydriden und den Tetracarbonsäuren ausgewählt ist, insbesondere den Tetracarbonsäuredianhydriden, deren polykondensierte aromatische Struktur sich von Anthracen, von Benzoanthracen, von Benzofluoranthen, von 1,12-Benzoperylen, von Benzopyren, von Chrysen, von Dibenzoacridin, von Dibenzoanthracen, Dibenzofuran, von Dibenzopyren, von Perylen oder von Phenanthren ableitet, wobei sie vorzugsweise aus dem Dianhydrid von 3,4,9,10-Perylentetracarbonsäure, 3,4,9,10-Perylentetracarbonsäure und deren Mischung ausgewählt ist und sie stärker bevorzugt zumindest das Dianhydrid von 3,4,9,10-Perylentetracarbonsäure umfasst.

10. Verfahren nach einem beliebigen der vorhergehenden Ansprüche, wobei die feststoffliche Mischung des Schrittes (i) zumindest wasserfreies oder als Hydrat vorliegendes, insbesondere als Hydrat vorliegendes, Lithiumhydroxid sowie zumindest das Dianhydrid von 3,4,9,10-Perylentetracarbonsäure umfasst.

11. Verfahren zur Herstellung einer Elektrode, welche als aktives Material mindestens ein Material vom Typ Alkalimetallcarboxylat umfasst, wobei es zumindest die folgenden Schritte umfasst:
(i) Bereitstellen einer feststofflichen Mischung, die mindestens eine Verbindung, welche eine Quelle von Alkaliionen ist, und mindestens eine polykondensierte aromatische Verbindung umfasst, welche aus den polykondensierten aromatischen (Poly)carbonsäuren und den (Poly)anhydriden polykondensierter aromatischer Säure ausgewählt ist;
(ii) Einwirkenlassen einer mechanischen Belastung, welche eine mechanochemische Wechselwirkung zwischen der/den Verbindung(en), welche eine Quelle von Alkaliionen ist/sind, und der/den polykondensierten aromatischen Verbindung(en) begünstigt, auf die feststoffliche Mischung des Schrittes (i), um das Alkalimetallcarboxylat zu bilden;
(iii) Gewinnen des Materials vom Typ Alkalimetallcarboxylat, insbesondere durch Sieben;
(iv) Herstellen einer Dispersion, die in einem wässrigen oder organischen Medium zumindest die Gesamtheit oder eine Teilmenge des Materials vom Typ Alkalimetallcarboxylat des Schrittes (iii) sowie möglicherweise ein(en) oder mehrere Zusatzstoff(e) elektronenleitender Art und/oder Bindemittel umfasst;
(v) Beschichten der Oberfläche eines Stromsammlers mit der Gesamtheit oder einer Teilmenge der Dispersion des Schrittes (iv); und
(vi) Verdampfen des wässrigen oder organischen Mediums, um die Elektrode zu bilden.

12. Verfahren nach dem vorhergehenden Anspruch, wobei die Schritte (i), (ii) und (iii) der Begriffsbestimmung in einem beliebigen der Ansprüche 1 bis 10 entsprechen.

## Claims

1. Process for the preparation of a material of electroactive alkali metal carboxylate type, comprising at least the stages consisting in:
(i) having available a solid mixture comprising at least one compound which is a source of alkaline ions and at least one polycondensed aromatic compound chosen from polycondensed aromatic (poly)carboxylic acids and polycondensed aromatic (poly)acid anhydrides,
(ii) subjecting said solid mixture of stage (i) to a mechanical stress favourable to the mechanochemical interaction of said compound(s) which is/are a source of alkaline ions and polycondensed aromatic compound(s) so as to form said alkali metal carboxylate, and
(iii) recovering said material of alkali metal carboxylate type, in particular by sieving.

2. Process according to Claim 1, in which said mechanochemical interaction is generated in stage (ii) by mechanical grinding of said solid mixture, in particular in a planetary mill or a bead mill, in particular in a planetary mill.

3. Process according to either one of the preceding claims, in which said solid mixture of stage (i), subjected to the mechanical stress, is devoid of a liquid medium and in particular of free water.

4. Process according to any one of the preceding claims, in which said solid mixture of stage (i) is a particulate mixture.

5. Process according to any one of the preceding claims, in which said solid mixture of stage (i) comprises at least one compound which is a source of alkaline ions chosen from compounds which are a source of lithium, sodium or potassium ions and their mixtures, and preferably comprises at least one compound which is a source of lithium ions.

6. Process according to any one of the preceding claims, in which said solid mixture of stage (i) comprises at least one compound which is a source of alkaline ions chosen from lithium, sodium or potassium carbonates and lithium, sodium or potassium hydroxides and their mixtures, preferably chosen from lithium, sodium or potassium hydroxides and more preferentially lithium hydroxide.

7. Process according to any one of the preceding claims, in which the solid mixture of stage (i) comprises at least one polycondensed aromatic compound chosen from polycondensed aromatic polycarboxylic acids and polycondensed aromatic polyacid anhydrides, preferably from polycondensed aromatic tetracarboxylic acid dianhydrides and polycondensed aromatic tetracarboxylic acids, and more preferentially from polycondensed aromatic tetracarboxylic acid dianhydrides.

8. Process according to any one of the preceding claims, in which said polycondensed aromatic compound is devoid of a hydroxyl function.

9. Process according to any one of the preceding claims, in which said solid mixture of stage (i) comprises at least one polycondensed aromatic compound chosen from tetracarboxylic acid dianhydrides and tetracarboxylic acids, in particular tetracarboxylic acid dianhydrides, the polycondensed aromatic structure of which derives from anthracene, benzoanthracene, benzofluoranthene, 1,12-benzoperylene, benzopyrene, chrysene, dibenzoacridine, dibenzoanthracene, dibenzofuran, dibenzopyrene, perylene or phenanthrene, preferably chosen from 3,4,9,10-perylenetetracarboxylic acid dianhydride, 3,4,9,10-perylenetetracarboxylic acid and their mixture, and more preferentially comprises at least 3,4,9,10-perylenetetracarboxylic acid dianhydride.

10. Process according to any one of the preceding claims, in which the solid mixture of stage (i) comprises at least anhydrous or hydrated, in particular hydrated, lithium hydroxide and at least 3,4,9,10-perylenetetracarboxylic acid dianhydride.

11. Process for the preparation of an electrode comprising, as active material, at least one material of alkali metal carboxylate type, comprising at least the following stages consisting in:
(i) having available a solid mixture comprising at least one compound which is a source of alkaline ions and at least one polycondensed aromatic compound chosen from polycondensed aromatic (poly)carboxylic acids and polycondensed aromatic (poly)acid anhydrides;
(ii) subjecting said solid mixture of stage (i) to a mechanical stress favourable to the mechanochemical interaction of said compound(s) which is/are a source of alkaline ions and polycondensed aromatic compound(s) so as to form said alkali metal carboxylate;
(iii) recovering said material of alkali metal carboxylate type, in particular by sieving;
(iv) preparing a dispersion comprising, in an aqueous or organic medium, at least all or part of said material of alkali metal carboxylate type of stage (iii), and optionally one or more electronically conductive additive(s) and/or binder(s);
(v) carrying out the deposition of all or part of said dispersion of stage (iv) at the surface of a current collector; and
(vi) evaporating said aqueous or organic medium so as to form said electrode.

12. Process according to the preceding claim, in which stages (i), (ii) and (iii) are as defined in any one of Claims 1 to 10.
